# EUROPÄISCHE PATENTSCHRIFT

(11) **EP 0 968 427 B1**
(45) Veröffentlichungstag und Bekanntmachung des Hinweises auf die Patenterteilung: **04.06.2003**
(21) Anmeldenummer: 98922615.4
(22) Anmeldetag: 11.03.1998
(51) Int. Cl.: G01N 33/564

(54) **VERFAHREN ZUR BESTIMMUNG VON SCHILDDRÜSEN-AUTOANTIKÖRPERN**
METHOD FOR DETECTING THYROID AUTOANTIBODIES
PROCEDE DE DETECTION D'AUTOANTICORPS DIRIGES CONTRE LA THYROIDE

(30) Priorität: 12.03.1997 DE 19710211
(43) Veröffentlichungstag der Anmeldung: 05.01.2000
(73) Patentinhaber: B.R.A.H.M.S Aktiengesellschaft, 16761 Hennigsdorf (DE)
(72) Erfinder: STRUCK, Joachim, D-12161 Berlin (DE)
(74) Vertreter: Andrae, Steffen, Dr.
(86) Internationale Anmeldenummer: EP9801401
(87) Internationale Veröffentlichungsnummer: WO98040742

(56) Entgegenhaltungen:
- GB-A- 2 265 713
- CHEMICAL ABSTRACTS, vol. 119, no. 19, 8. November 1993 Columbus, Ohio, US; abstract no. 201596, XP002074219 & J. RUF ET AL.: "Significance of thyroglobulin antibodies cross-reactive with thyroperoxidase (TGPO antibodies) in individual patients and immunized mice." CLINICAL AND EXPERIMENTAL IMMUNOLOGY, Bd. 92, Nr. 1, 1993, Seiten 65-72, Oxford UK
- CHEMICAL ABSTRACTS, vol. 111, no. 19, 6. November 1989 Columbus, Ohio, US; abstract no. 172028, XP002074222 & K. BEEVER ET AL: "Highly sensitive assays of autoantibodies to thyroglobulin and to thyroid peroxidase." CLINICAL CHEMISTRY, Bd. 35, Nr. 9, 1989, Seiten 1949-1954, Winston-Salem NC USA

## Beschreibung

Die Erfindung betrifft ein neues Verfahren zur Bestimmung von Autoantikörpern, die gegen Schilddrüsen-Autoantigene gerichtet sind und die im folgenden kurz als "Schilddrüsen-Autoantikörper" bezeichnet werden.

Unter den Erkrankungen, bei denen es zu Störungen der normalen Funktion und/oder zu mikroskopisch oder optisch wahrnehmbaren Veränderungen der Schilddrüse kommt, nehmen solche, die den Charakter von Autoimmunerkrankungen aufweisen, eine wichtige Rolle ein. Die bekanntesten Erkrankungen der Schilddrüse, die zu den Autoimmunerkrankungen gerechnet werden müssen, sind insbesondere der Morbus Basedow (englisch: Grave's disease), die Hashimoto-Thyreoiditis, und die primäre Hypothyreose. Als weitere derartige Erkrankungen sind zu nennen die atrophische Autoimmunthyreoiditis, das primäre Myxödem, die asymptomatische Thyreoiditis, die postpartale Thyreoiditis und die neonatale Hypothyreose, wobei, je nach dem klinischen Bild, unterschiedliche Bezeichnungen für gleiche oder gleichartige Erkrankungen verwendet werden.

Der differentialdiagnostische Nachweis, daß die beobachtete Schilddrüsenerkrankung zu den Autoimmunerkrankungen gehört, wird dadurch geführt, daß man bei dem Patienten Autoantikörper nachweist. Der Nachweis dieser Autoantikörper gestattet somit in vielen Fällen die differentialdiagnostische Abgrenzung von Autoimmunerkrankungen gegenüber anderen Schilddrüsenkrankheiten (vgl. Pfannenstiel, P. & Saller, B. in "Schilddrüsenkrankheiten - Diagnose und Therapie", Berliner Medizinische Verlagsanstalt, 1993; oder Ziegler, R., Pickardt, C.R., Willig, R.-P. in "Rationelle Diagnostik in der Endokrinologie", Georg Thieme Verlag, 1993). Die bei Schilddrüsen-Autoimmunerkrankungen auftretenden bekannten Autoantikörper gehören drei unterschiedlichen Typen an, die sich durch die als Autoantigene fungierenden Schilddrüsen-Proteine unterscheiden. Die drei Schilddrüsen-Autoantigene sind der TSH-Rezeptor, die thyreoidale Peroxidase (TPO, identisch mit dem früheren sogenannten mikrosomalen Antigen) und Thyreoglobulin (Tg) (Dawe, K., Hutchings, P., Champion, B., Cooke, A., Roitt, I., "Autoantigens in Thyroid diseases", Springer Semin. Immunopathol. 14, 285-307, 1993). Autoantikörper gegen den TSH-Rezeptor treten dabei spezifisch beim Morbus Basedow auf, nicht jedoch bei anderen Autoimmunerkrankungen der Schilddrüse (Pfannenstiel & Saller, a.a.O.). Daneben sind beim Morbus Basedow in vielen Fällen auch Autoantikörper gegen die beiden anderen Autoantigene, d.h. gegen TPO und Tg, mit relativ geringer Sensitivität nachweisbar. Für die Diagnose auf Morbus Basedow spielt der Nachweis von Autoantikörpern gegen TPO und/oder Tg jedoch angesichts der Relevanz und Spezifität des Nachweises von Autoantikörpern gegen den TSH-Rezeptor keine wesentliche Rolle.

Eine Bestimmung von anti-TPO-Autoantikörpern und anti-Tg-Autoantikörpern erfolgt insbesondere bei einem Verdacht auf eine Autoimmunthyreoiditis. Bei der Autoimmunthyreoiditis vom Typ Hashimoto und der atrophischen Autoimmunthyreoiditis finden sich sehr häufig anti-TPO-Autoantikörper, jedoch keine Autoantikörper gegen den TSH-Rezeptor. Häufig wird das Auftreten von anti-TPO-Autoantikörpern durch anti-Tg-Autoantikörper begleitet, wobei es jedoch Fälle gibt, bei denen ausschließlich anti-Tg-Autoantikörper, nicht jedoch anti-TPO-Autoantikörper nachweisbar sind (Meng, W. in "Schilddrüsenerkrankungen", Gustav Fischer Verlag, 1992; Pfannenstiel & Saller, a.a.O; Ziegler et al., a.a.O.). Da es somit Fälle einer Autoimmunthyreoiditis gibt, bei denen keine anti-TPO-Autoantikörper nachweisbar sind, wird die Bestimmung von anti-Tg-Autoantikörpern zusätzlich zur Bestimmung von anti-TPO-Autoantikörpern empfohlen (Feldt-Rasmussen, U. "Analytical and clinical performance goals for testing autoantibodies to thyroperoxidase, thyroglobulin, and thyrotropin receptor", Clinical Chemistry 42:1, 160-163, 1996). Während es neben der Autoimmunthyreoiditis vom Typ Hashimoto weitere Schilddrüsenkrankheitsbilder gibt, die Autoimmunkrankheiten darstellen und bei denen anti-TPO- oder anti-Tg-Autoantikörper erhöht sein können (primäres Myxödem, asymptomatische Thyreoiditis, postpartale Thyreoiditis, neonatale Hypothyreose), gibt es nur eine Indikation, bei der anti-TPO-Autoantikörper nicht neben anti-Tg-Autoantikörpern auftreten und zur Messung empfohlen werden, nämlich das Schilddrüsenkarzinom (Feldt-Rasmussen, a.a.O.). Beim Schilddrüsenkarzinom werden große Mengen Thyreoglobulin aus den Schilddrüsenzellen freigesetzt, was dazu führen kann, daß sich Autoantikörper gegen Tg bilden. Normalerweise wird zum Nachweis eines Schilddrüsenkarzinoms daher Thyreoglobulin (Tg) bestimmt. Die Tg-Bestimmung kann jedoch durch die Anwesenheit von anti-Tg-Autoantikörpern gestört werden, weshalb zur Absicherung der Meßergebnisse der Tg-Bestimmung eine sogenannte Wiederfindungsmessung durchgeführt werden muß. Zur Absicherung der Befunde bei einem gestörten Tg-Nachweis kann es in Einzelfällen interessant sein, die entsprechende biologische Probe auf eine Anwesenheit von anti-Tg-Autoantikörpern zu prüfen.

Das dominierende Verfahren zur Bestimmung von Autoantikörpern bei Verdacht auf eine Autoimmunthyreoiditis ist die Bestimmung von anti-TPO-Autoantikörpern. Die Verläßlichkeit derartiger Bestimmungen im Hinblick auf ihre diagnostische Sensitivität wird jedoch diskutiert (Haagen, U. & Bergmann, A., "Besondere Aspekte der Qualitätssicherung der Komponente Autoantigen bei Fertigreagenzien für die Schilddrüsenautoimmundiagnostik", Abstract von der "Jahrestagung der Sektion Schilddrüse der Deutschen Gesellschaft für Endokrinologie, 23.-25.11.1995, Hannover, S. 15-22): Bei bestimmten immunologischen Bestimmungsverfahren für anti-TPO-Autoantikörper wird das Antigen TPO als Assaykomponente in einer Form eingesetzt, bei der möglicherweise nicht mehr alle von Autoantikörpern erkannten Epitope vorhanden sind, wobei insbesondere konformationelle Epitope fehlen können. Das kann der Fall sein, wenn ein gentechnisch erzeugtes rekombinantes Antigen verwendet wird, wenn das Antigen gereinigt und dabei konformationell gestreßt worden ist, wenn das Antigen direkt auf einer Festphase immobilisiert wird und/oder wenn das Antigen unter rigiden Bedingungen markiert wird. Auch abhängig vom Assaydesign kann es vorkommen, daß bestimmte signifikante anti-TPO-Autoantikörper nicht nachgewiesen werden (Mariotti, S. et al., "False negative results observed in anti-thyroid peroxidase autoantibody determination by competitive radioimmunoassays using monoclonal antibodies", Eur. J. Endocrinol. 130, 552-558, 1994).

Nachdem somit Fälle bekannt sind, bei denen die alleinige Bestimmung von anti-TPO-Autoantikörpern ein unrichtiges negatives Ergebnis liefert, wird empfohlen, zur diagnostischen Absicherung eine zusätzliche zweite Bestimmung, nämlich von anti-Tg-Autoantikörpern, durchzuführen.

Trotz der Vorteile, die eine zusätzliche anti-Tg-Bestimmung erbringt, wird angesichts des zunehmenden Kostendrucks im Gesundheitswesen diskutiert, bei der Diagnose der Hashimoto-Thyreoiditis auf eine zusätzliche anti-Tg-Bestimmung zu verzichten, obwohl anerkannt wird, daß dadurch die Gefahr von Fehldiagnosen erhöht wird (Nordyke, R.A. et al., "The superiority of antimicrosomal over antithyroglobulin antibodies for detecting Hashimoto's thyroiditis", Arch. Intern. Med. 153, 862-865, 1993).

Unter diesen Umständen ist es Aufgabe der vorliegenden Erfindung, ein Verfahren zur Bestimmung von Schilddrüsen-Autoantikörpern im Rahmen der Differentialdiagnostik von Schilddrüsen-Erkrankungen zu schaffen, das einerseits nur den relativ geringen Aufwand einer Einzelbestimmung erfordert, jedoch eine diagnostische Sensitivität aufweist, die wenigstens gegenüber der Bestimmung nur einer Sorte von Autoantikörpern das Risiko unrichtiger negativer Ergebnisse vermindert, sowie ferner mindestens eine Möglichkeit für die erfolgreiche technische Durchführung eines solchen Verfahrens aufzuzeigen.

Diese Aufgabe wird durch ein Verfahren gemäß Oberbegriff von Patentanspruch 1 gelöst, das dadurch gekennzeichnet ist, daß man ein immundiagnostisches Bestimmungsverfahren anwendet, bei dem pro Bestimmung ein Meßsignal gewonnen wird, das summarisch die Anwesenheit und Menge von mindestens zwei Antikörpern, zu denen anti-TPO-Auto-antikörper und anti-Tg-Autoantikörper gehören, in der Probe repräsentiert.

Zum gegenwärtigen Zeitpunkt bevorzugte technische Ausführungsformen eines solchen Verfahrens sind in den Unteransprüchen 2 bis 5 näher definiert. Anspruch 6 definiert die Indikationen genauer, bei denen, wenn ein entsprechender Verdacht besteht, eine Durchführung des erfindungsgemäßen Verfahrens vorteilhaft ist.

Der Erfindung lag die Idee zugrunde, systematisch zu prüfen, inwieweit eine parallele Erfassung der beiden genannten Arten von Autoantikörpern nachweisbare Vorteile gegenüber einer Einzelbestimmung nur einer Art von Autoantikörpern bietet, und dann festzustellen, ob es möglich ist, zwei unterschiedliche Arten von Autoantikörpern, nämlich anti-TPO-Autoantikörper und anti-Tg-Autoantikörper, in biologischen Proben, insbesondere Serumproben von Patienten, im Rahmen einer einzigen Bestimmung gleichzeitig und ohne zwischen den beiden Arten von Autoantikörpern zu differenzieren zu erfassen. Im Falle der Möglichkeit einer zuverlässigen summarischen Bestimmung beider Autoantikörper war dann ferner festzustellen, welche diagnostische Sensitivität und Spezifität eine solche summarische Bestimmung im Vergleich mit isolierten Bestimmungen von anti-TPO- und anti-Tg-Autoantikörpern hat.

Zur Prüfung der obigen Idee wurde ein klinische Untersuchung durchgeführt, bei der jeweils nebeneinander die Konzentrationen von anti-TPO-Autoantikörpern und anti-Tg-Autoantikörpern in Serumproben verschiedener Gruppen von Patienten, die an Schilddrüsenerkrankungen litten, auf herkömmliche Weise unter Verwendung der handelsüblichen Tests DYNOtest® anti-TPOₙ und DYNOtest® anti-Tgₙ der Anmelderin B.R.A.H.M.S Diagnostica GmbH bestimmt wurden. Die Ergebnisse der Paare von Einzelmessungen wurden dazu verwendet, zu ermitteln, welche Konzentrationen an Autoantikörpern erhalten werden, wenn man die ermittelten Konzentrationen rechnerisch summiert. (Für die rechnerische Auswertung wurde eine direkte Aufsummierung gewählt, wobei grundsätzlich jedoch auch eine geeignete Umkalibrierung der Meßwerte möglich ist.) Die handelsüblichen Tests DYNOtest® anti-TPOₙ und DYNOtest® anti-Tgₙ geben jeweils Grenzwerte (Cutoff-Werte) für die erhaltenen Meßergebnisse an, deren Überschreiten eine untersuchte Probe als Autoantikörper positiv kennzeichnet. Für die Bestimmung von anti-TPO-Autoantikörpern und von anti-Tg-Autoantikörpern wird dabei jeweils ein Wert von 60 mU/l angegeben. Für eine gleichzeitige Berücksichtigung der Anwesenheit beider Arten von Autoantikörpern muß ein neuer Cutoff-Wert festgelegt werden, der im Rahmen der Untersuchung 73 mU/l betrug.

Bei der genannten gemeinsamen rechnerischen Auswertung der Ergebnisse der Einzelbestimmungen der anti-TPO-Autoantikörper und anti-Tg-Autoantikörper ergab sich überraschend, daß sich durch eine rechnerische Kombination der Einzelmessungen eine höhere diagnostische Sensitivität für die relevanten Patientengruppen erreichen läßt, als bei isolierten Bestimmungen von anti-TPO-Autoantikörpern oder anti-Tg-Autoantikörpern. Bei der gemeinsamen rechnerischen Auswertung der Ergebnisse der Einzelmessungen wurde festgestellt, daß im wesentlichen alle Proben, die in Einzelbestimmungen entweder anti-TPO-Autoantikörperpositiv oder anti-Tg-Autoantikörper-positiv gemessen wurden, auch bei einer summarischen Auswertung in jedem der Fälle positiv gemessen wurden. Ferner zeigte sich, daß die Meßergebnisse auch bei einer gemeinsamen Auswertung spezifisch waren, d.h. es wurden keine fehlerhaften Ergebnisse für das Normalkollektiv erhalten. Die Ergebnisse für die verschiedenen Patientengruppen sind in Tabelle 1 zusammengefaßt. In der Tabelle 1 enthält die Spalte SUMME die Ergebnisse einer summarischen Auswertung der paarweisen Einzelmessungen.

Bei einer gemeinsamen Erfassung der anti-TPO-Autoantikörper-Konzentrationen und der anti-Tg-Autoantikörper-Konzentrationen wird somit eine diagnostische Sensitivität erhalten, die besser ist als die von isolierten Messungen einer der genannten Konzentrationen, während gleichzeitig die Spezifität unbeeinträchtigt bleibt.

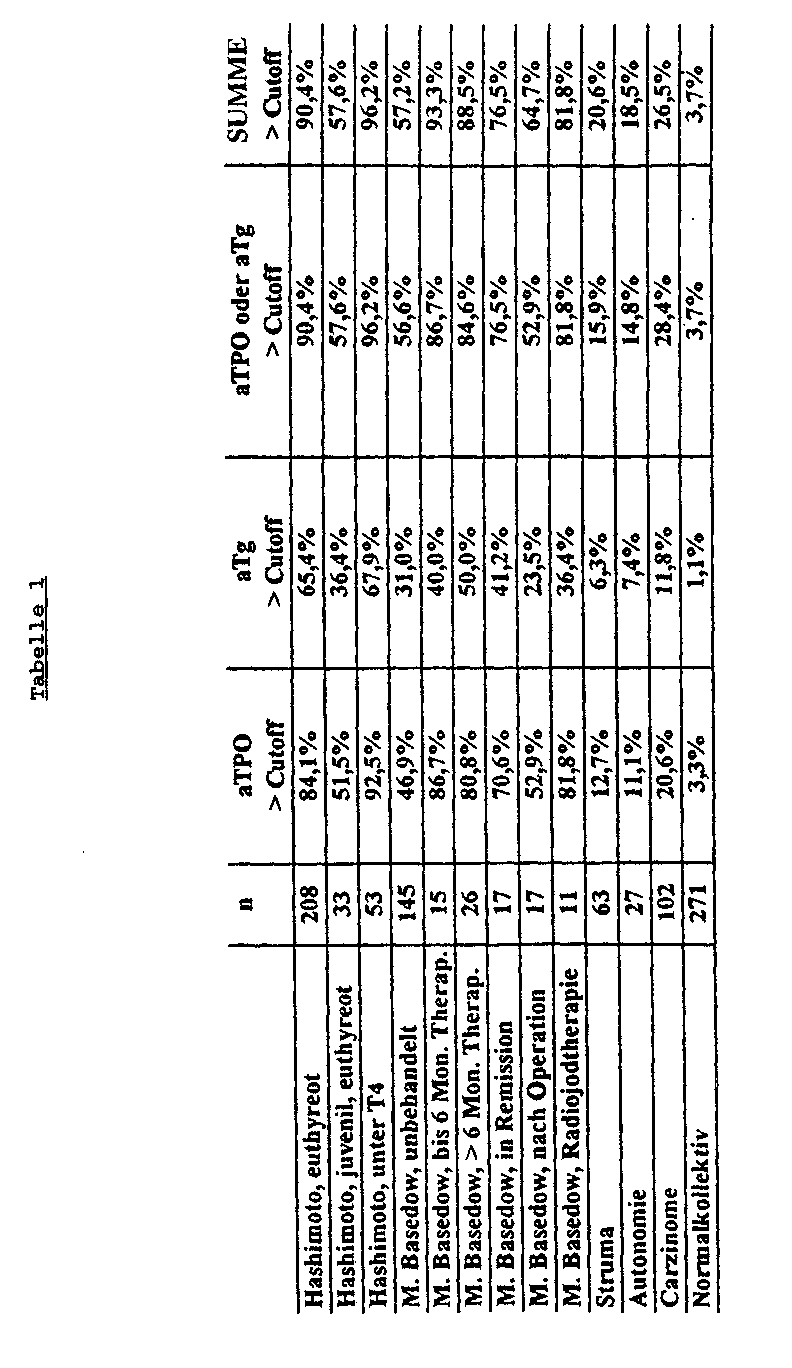

Aus den Ergebnissen der rechnerischen Auswertung der beschriebenen Versuchsreihen wurde der Schluß gezogen, daß es nicht erforderlich ist, zur Gewinnung zuverlässiger Meßergebnisse zwei Einzelbestimmungen durchzuführen, sondern daß dann, wenn es technisch möglich ist, eine einzige summarische Bestimmung beider Parameter (Konzentrationen der anti-TPO-Autoantikörper und anti-Tg-Autoantikörper) durchzuführen, summarische Meßergebnisse erhalten werden müßten, die Einzelmessungen bezüglich der diagnostischen Sensitivität überlegen sind, während gleichzeitig die Spezifität unbeeinträchtigt bleibt. Unter Kostenaspekten und auch Handhabungsaspekten sind die Vorteile einer summarischen Einzelmessung eklatant: Es müssen nicht zwei Tests durchgeführt werden, sondern es genügt ein einziger. Das senkt erheblich die Kosten bei Material, Personal, Erstattung, führt zu einer besseren Handhabbarkeit und, wegen der Verminderung der Anzahl der durchzuführenden Messungen und dem damit verminderten Anfall an zu entsorgenden Meßlösungen, zu einer umweltfreundlicheren Bestimmung. Eine Durchsicht der Literatur hat bestätigt, daß keine Indikation von diagnostischer oder therapeutischer Bedeutung bekannt ist, die es erfordert, zu differenzieren, ob anti-TPO-Autoantikörper oder anti-Tg-Autoantikörper erhöht sind. Die summarische Bestimmung führt somit gegenüber der bisherigen Praxis zu keinem Informationsverlust.

Zur Prüfung der Frage, ob die aus einer gemeinsamen rechnerischen Auswertung von Einzelbestimmungen gezogenen Schlüsse auch dann richtig sind, wenn man versucht, beide Parameter summarisch im Rahmen einer einzigen Bestimmung zu erfassen, d.h. um sicher zu stellen, daß es nicht zu einer wechselseitigen Störung der Bestimmungen der zwei Arten von Autoantikörpern kommt und daß ein summarisches Meßsignal gewonnen werden kann, das die gesamte Autoantikörperkonzentration repräsentiert und die gewünschte Selektivität und Sensitivität des Tests garantiert, wurde der im nachfolgenden Beispiel beschriebene summarische Test durchgeführt.

Bei Verfahren zur summarischen Bestimmung beider Arten von Autoantikörpern wird grundsätzlich so vorgegangen, daß das angewandte immundiagnostische Bestimmungsverfahren wenigstens einen Verfahrensschritt aufweist, bei dem eine ggf. verdünnte flüssige Probe mit wenigstens zwei selektiven Reagenzien umgesetzt wird, von denen eines eine selektive Bindung mit anti-TPO-Autoantikörpern und das andere eine selektive Bindung mit anti-Tg-Autoantikörpern eingeht, wobei die wenigstens zwei selektiven Reagenzien entweder zur selektiven Abtrennung der anti-TPO-Autoantikörper und der anti-Tg-Autoantikörper vom Rest der flüssigen Probe oder zu deren selektiver Markierung vor oder nach ihrer Abtrennung von der flüssigen Phase eingesetzt werden.

Das im Beispiel angewandte Verfahren läßt sich in allgemeiner Form beschreiben als Verfahren, das die Schritte umfaßt:
- Binden der in der flüssigen Probe vorhandenen Antikörper, einschließlich der zu bestimmenden Autoantikörper, mittels eines festphasengebundenen unspezifischen Binders an eine feste Phase,
- gemeinsame oder sequenzielle Zugabe von zwei verschiedenen Tracer-Reagenzien, die den gleichen Markierungsbestandteil enthalten, wobei eines der Tracer-Reagenzien spezifisch alle gebundenen anti-TPO-Autoantikörper markiert und das andere der Tracer-Reagenzien spezifisch alle gebundenen anti-Tg-Autoantikörper markiert,
- Abtrennen der festen Phase von der flüssigen Reaktionsmischung und
- Messung der an die feste Phase gebundenen Markierung und Gewinnung eínes Meßsignals, das die Summe der Mengen der anti-TPO-Autoantikörper und anti-Tg-Autoantikörper in der Probe repräsentiert.

Als Tracer-Reagens zur Markierung von anti-TPO-Autoantikörpern verwendet man, wie im Patent DE 41 20 412 beschrieben, einen cruden Extrakt aus humanen Schilddrüsen, der humane TPO in nativer, undenaturierter Form enthält, und in dem die humane TPO mittels markierter Antikörperfragmente selektiv markiert ist oder markiert werden kann, ohne daß ihre Bindungsfähigkeit gegenüber anti-TPO-Autoantikörpern beeinträchtigt ist.

### Beispiel für die summarische Bestimmung von anti-Tg- und anti-TPO-Autoantikörpern

Es wurde eín neues Testsystem geschaffen, bei dem die Komponenten zweier kommerzieller Immunpräzipitationstests der Anmelderin, der Firma B.R.A.H.M.S Diagnostica GmbH, verwendet wurden (HENNINGtest® anti-Tg und HENNINGtest® anti-TPO).

Beide für die Einzelbestimmungen von anti-Tg-Autoantikörpern und anti-TPO-Autoantikörpern ausgelegten Tests arbeiten mit einer Protein A-Suspension als Festphase. Die in der Probe vorhandenen Antikörper werden unspezifisch an die Protein A-Suspension gebunden. Bei den Einzelbestimmungen werden dann die gesuchten Autoantikörper mit spezifischen Tracern selektiv radioaktiv markiert, wobei zur Markierung von anti-Tg-Autoantikörpern mit radioaktiv markiertem Tg gearbeitet wird, während anti-TPO-Autoantikörper unter Verwendung eines kruden Schilddrüsenmembranextrakts, der natives TPO enthält, das mit Hilfe eines radioaktiv markierten Fab-Fragments eines monoklonalen anti-TPO-Antikörpers selektiv affinitätsmarkiert ist, nachgewiesen werden.

Im Rahmen der Untersuchung wurden 130 verschiedene Patientenseren mit verschiedenen Konzentrationen an anti-TPO-Autoantikörpern und anti-Tg-Autoantikörpern in Einzelbestimmungen gemäß den Arbeitseinleitungen der beiden genannten handelsüblichen Tests vermessen.

Zusätzlich wurde ein Kombinationstest durchgeführt, der einer summarischen Bestimmung von anti-TPO-Autoantikörpern und anti-Tg-Autoantikörpern dienen sollte und der nur ein einziges Meßsignal liefert.

Dazu wurden jeweils 50 µl der Probe, die gemäß den Arbeitseinleitungen der obigen Tests vorverdünnt war, mit 50 µl Protein A-Suspension versetzt. Anschließend wurden 50 µl des Tracers zum Nachweis von anti-TPO-Autoantikörpern zugesetzt, und nach 15 min wurden der gleichen Probe 50 µl Tracer zum anti-Tg-Autoantikörpernachweis zugesetzt. Nach Zugabe der beiden Tracer wurde 1 h bei Raumtemperatur inkubiert, mit 1 ml Puffer aufgefüllt, und anschließend wurde die suspendierte Festphase durch Zentrifugation von der Testflüssigkeit getrennt, und es wurde die an die Festphase gebundene Radioaktivität gemessen.

Die erhaltenen Meßergebnisse sind in Tabelle 2 zusammengestellt. In der Tabelle 2 sind Ergebnisse der Messungen der an die abgetrennte Festphase gebundenen Radioaktivitäten, angegeben in cpm, und die daraus errechneten Autoantikörperkonzentrationen (U/ml) angegeben. Ferner enthält Tabelle 2 die aus den Summen der Einzelbestimmungen errechneten Gesamt-Autoantikörperkonzentrationen (Spalte Summe aTPO+aTg). Die bei der gemeinsamen Bestimmung im Kombinationstest (Spalte COMBI) gemäß dem beschriebenen Beispiel erhaltenen Signale wurden auf die errechneten Summen der Einzelbestimmungen bezogen. Schraffiert sind diejenigen Bestimmungen, die klinisch als Autoantikörperpositiv bewertet werden, wenn für die Einzelbestimmungen jeweils die angegebenen Cutoff-Werte von 100 U/ml zugrunde gelegt werden, und für den Kombinationstest ein Cutoff entsprechend einem Radioaktivitätssignal von 2900 cpm zugrunde gelegt wird.

Die Auswertung zeigt, daß im Kombinationstest für die einzelnen Proben annähernd ideal diejenigen Radioaktivitätssignale gemessen wurden, wie sie sich rechnerisch ergeben, wenn man die Radioaktivitätssignale separater anti-TPO- und anti-Tg-Bestimmungen addiert. Dieses Ergebnis beweist, daß eine summarische Messung von anti-Tg- und anti-TPO-Autoantikörpern technisch möglich ist.

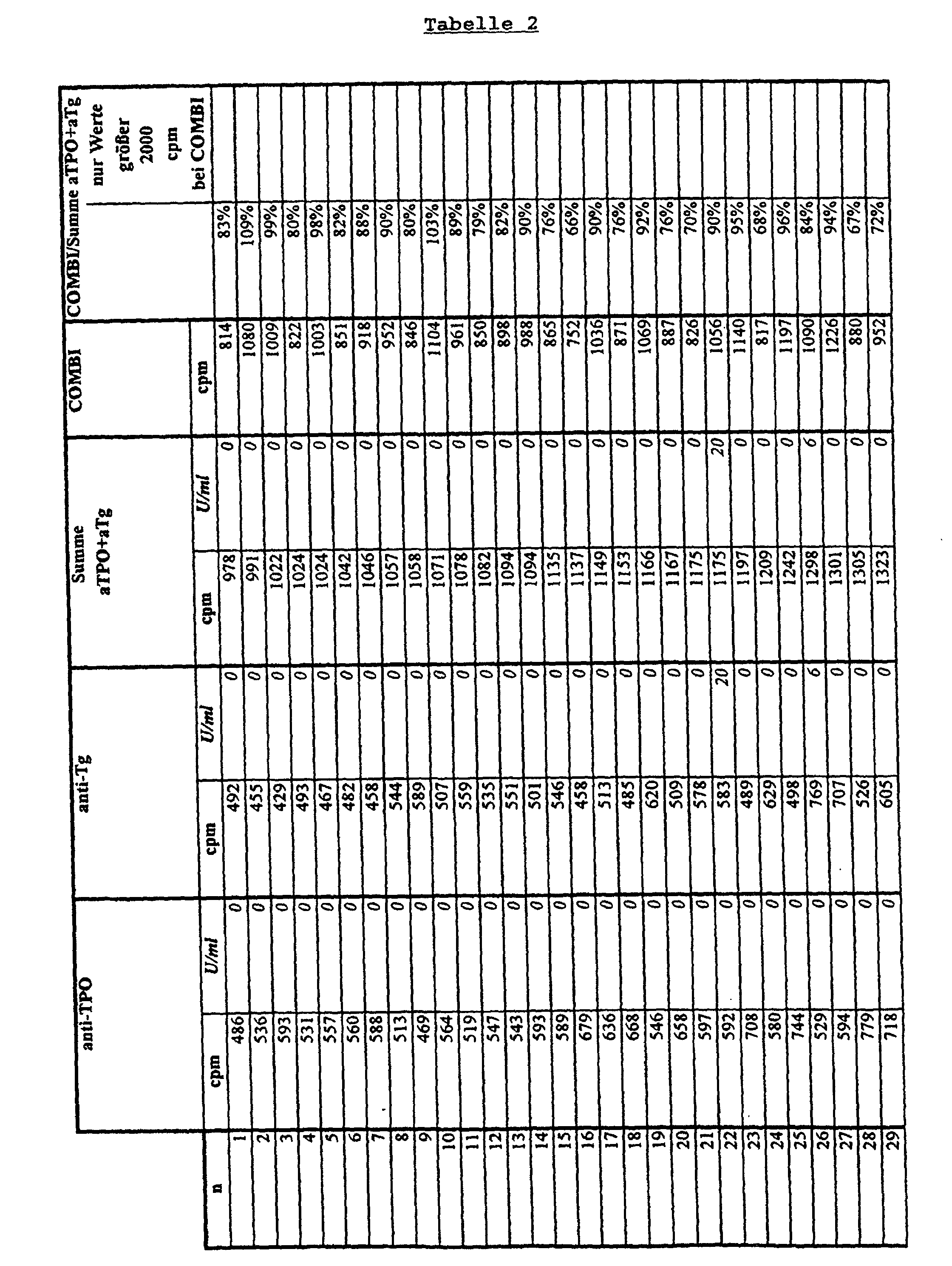

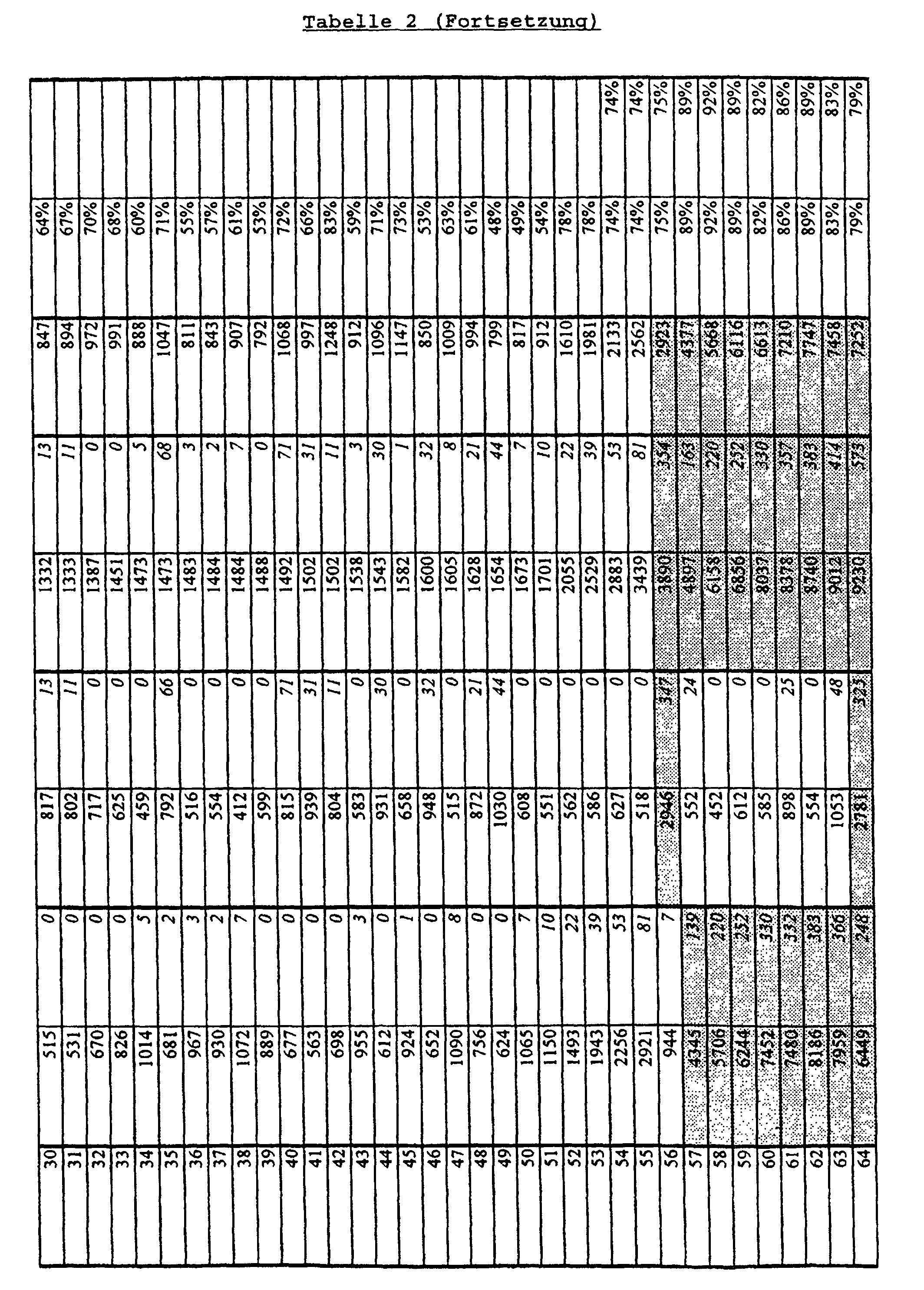

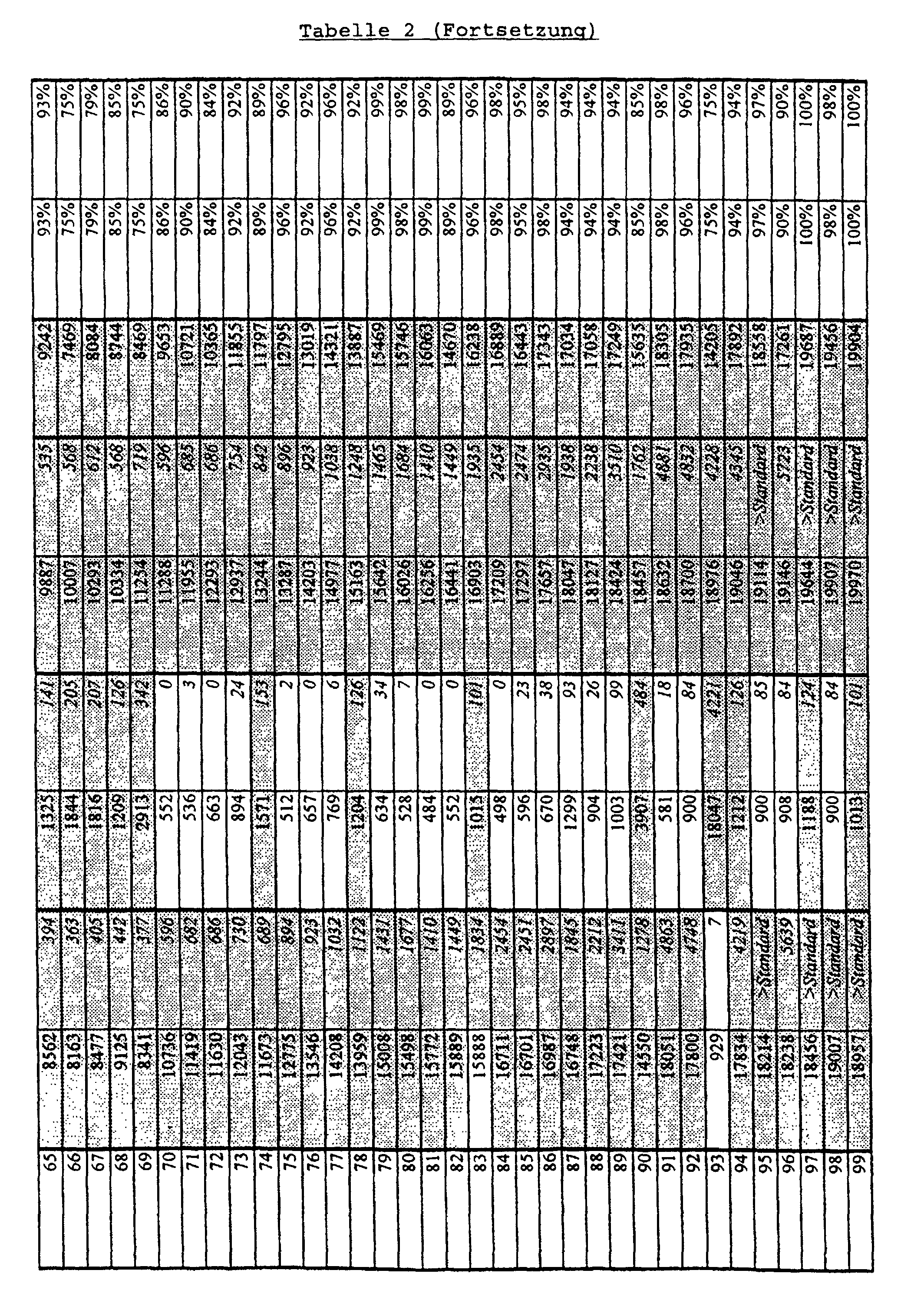

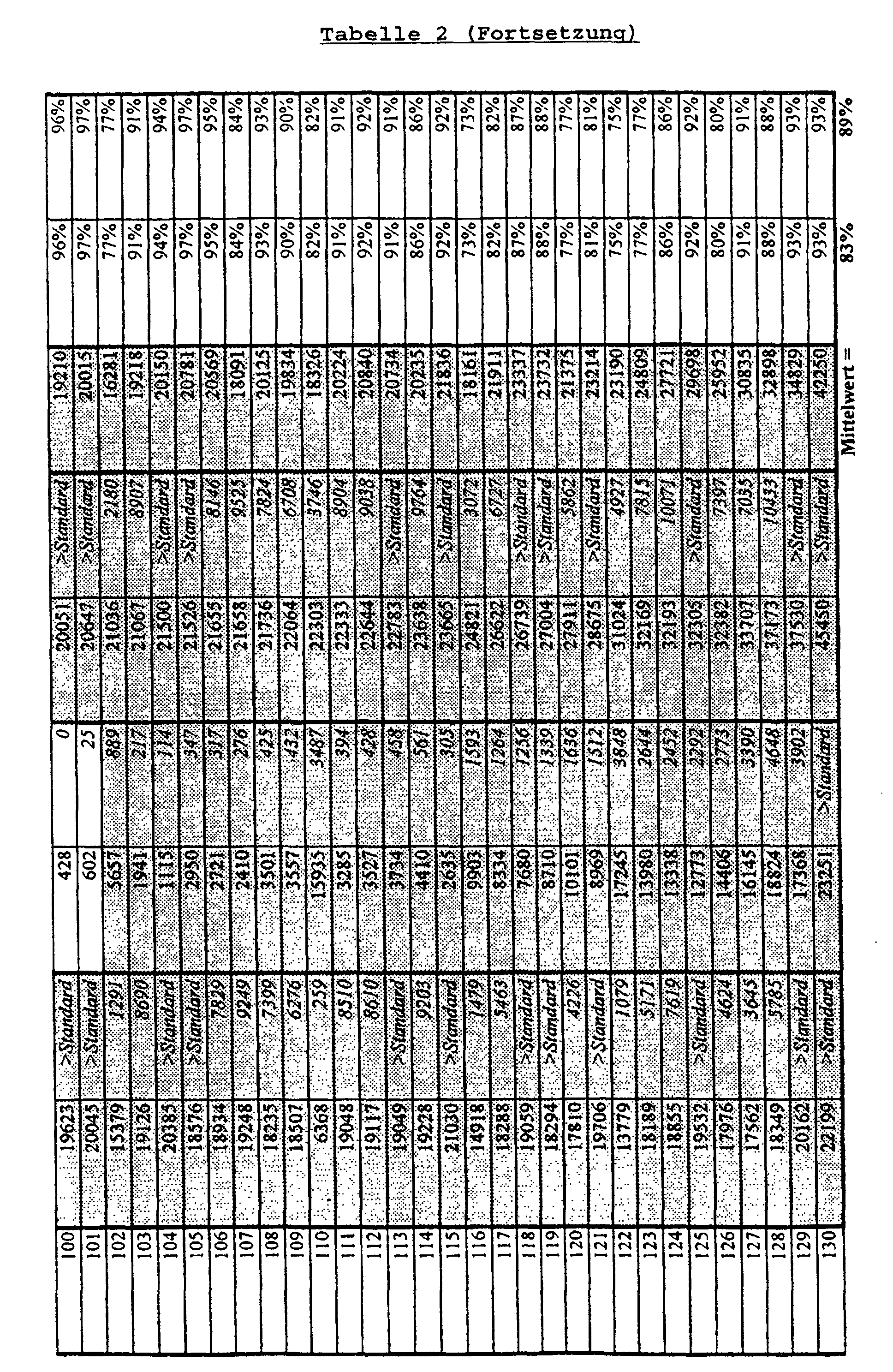

Die beschriebene summarische Messung unter Verwendung der Komponenten der bekannten Immunpräzipitationstests stellt nur eine Möglichkeit der technischen Umsetzung der summarischen Bestimmung der beiden Arten von Autoantikörpern dar. Die Kombination anderer Assaydesigns erscheint in ähnlicher Weise möglich, und Tests, die nach anderen Assayprinzipien arbeiten, sollen von dem erfindungsgemäßen Verfahren ebenfalls umfaßt werden. Insbesondere sind dabei zu nennen:

Ein kombinatorischer Assay zur summarischen Bestimmung von anti-TPO- und anti-Tg-Autoantikörpern nach dem kompetitiven Prinzip, wie es beschrieben ist z.B. in dem Patent DE 41 20 412. Dabei kann man mit einer gemischten Festphase arbeiten, an die sowohl selektive anti-TPO-Antikörper als auch anti-Tg-Antikörper gebunden sind. Wenn man mit einem Tracergemisch arbeitet, das sowohl radioaktiv markiertes Tg als auch TPO enthält, äußert sich die Anwesenheit der gesuchten Autoantikörper in der Probe im Vergleich mit einer Autoantikörper-freien Probe als Verminderung der an die Festphase gebundenen Tracermenge. Gegebenenfalls müßte man dann, wenn in den verwendeten TPO-Präparationen Tg vorhanden ist, dieses durch bekannte Methoden, wie z.B. Affinitätschromatographie oder andere chromatographische Methoden, abtrennen. Die gewünschte technische Sensitivität läßt sich über geeignete Mengen an Festphasenantikörpern und Tracer einstellen, und die Zuverlässigkeit der Meßergebnisse wird durch geeignete Kalibrierungsmaßnahmen gewährleistet.

Auch ein kombinatorischer Assay mit immobilisierten Antigenen ist realisierbar. Dabei würden die Antigene Tg und TPO direkt oder über geeignete vorimmobilisierte anti-Tg- und anti-TPO-Antikörper an eine Festphase immobilisiert. Die nach Umsetzung mit der Patientenprobe gebundenen anti-Tg- und anti-TPO-Autoantikörper können zur Gewinnung eines summarischen Meßsignals z.B. mit markiertem Protein A oder markiertem antihuman-IgG nachgewiesen werden.

Ferner ist ein kombinatorischer Assay denkbar, der sich die Bivalenz der zu messenden Antikörper zunutze macht. Dazu könnten die Antigene Tg und TPO direkt oder über geeignete vorimmobilisierte selektive anti-Tg- und anti-TPO-Antikörper an eine Festphase immobilisiert werden. Die nach der Umsetzung mit der Patientenprobe gebundenen anti-Tg- und anti-TPO-Autoantikörper können dann durch ein Tracergemisch nachgewiesen werden, das sowohl radioaktiv markiertes Tg als auch radioaktiv markiertes TPO enthält. Auch in diesem Falle könnten in den TPO-Präparationen vorhandene Anteile an Tg durch die oben erwähnten bekannten chromatographischen Methoden abgetrennt werden.

Weitere an sich bekannte Assaydesigns sind denkbar. Die oben aufgezählten Assayvarianten wurden deshalb hervorgehoben, da sie gegenwärtig für die routinemäßige Bestimmung von anti-Tg-Autoantikörpern und anti-TPO-Autoantikörpern von besonderer Bedeutung sind.

Bei den oben beschriebenen Assayvarianten wurde ferner davon ausgegangen, daß die als Tracer verwendeten Reagenzien den gleichen Markierungsanteil enthalten, damit ein summarisches einziges Meßsignal erhalten werden kann. Das schließt es jedoch nicht aus, daß mindestens eines der Reagenzien evtl. noch einen weiteren Markierungsbestandteil enthält, der es ermöglicht, unter Verwendung der gleichen Meßlösung einen der zu bestimmenden Autoantikörper neben der summarischen Bestimmung auch einzeln zu messen. Bei einem Assaysystem, bei dem die Tracer unterschiedliche Markierungsbestandteile aufweisen, ist es in der Regel nicht möglich, ein summarisches Meßsignal zu erhalten. Solche Assays können, bei einer geeigneten Tracerwahl, gegenüber den herkömmlichen zwei getrennen Messungen jedoch noch den Vorteil aufweisen, daß zur Herstellung der Meßlösung nur einmal pipettiert werden muß und zwei Meßergebnisse mit einem einzigen Ansatz gewonnen werden können.

In der vorliegenden Beschreibung wird nur die gemeinsame Erfassung von zwei bestimmten Autoantikörpern, nämlich anti-TPO-Autoantikörpern und anti-Tg-Autoantikörpern, in näheren Einzelheiten beschrieben. Es liegt jedoch im Bereich der vorliegenden Erfindung, das Verfahren auf Fälle auszudehnen, bei denen im Rahmen der summarischen Messung ein oder mehrere weitere Antikörper miterfaßt werden, seien es Autoantikörper, deren zusätzliche Erfassung diagnostisch wertvoll ist, oder seien es irgendwelche zugesetzten Antikörper, deren Anwesenheit in den Meßlösungen zu einem konstanten Basissignal führt, das eine Gegenkontrolle der Korrektheit der Messungen ermöglicht oder den Meßbereich verschiebt.

## Patentansprüche

1. Verfahren zur Bestimmung von Schilddrüsen-Autoantikörpern in einer aus einem Patienten gewonnenen biologischen Probe im Rahmen der Differentialdiagnostik von Erkrankungen, die mit Veränderungen der Schilddrüse und/oder Störungen der normalen Schilddrüsenfunktion verbunden sind, wobei die Bestimmung der Anwesenheit und/oder Menge der Schilddrüsen-Autoantikörper in der Probe unter Anwendung eines immundiagnostischen Bestimmungsverfahrens erfolgt,
**dadurch gekennzeichnet, daß** man ein immundiagnostisches Bestimmungsverfahren anwendet, bei dem ein Meßsignal gewonnen wird, das summarisch die Anwesenheit und Menge von mindestens zwei Antikörpern, zu denen anti-TPO-Autoantikörper und anti-Tg-Autoantikörper gehören, in der Probe repräsentiert.

2. Verfahren nach Anspruch 1, **dadurch gekennzeichnet, daß** das immundiagnostische Bestimmungsverfahren den Schritt einer Umsetzung einer ggf. verdünnten flüssigen Probe mit wenigstens zwei selektiven Reagenzien umfaßt, von denen eines eine selektive Bindung mit anti-TPO-Autoantikörpern und das andere eine selektive Bindung mit anti-Tg-Autoantikörpern eingeht, wobei die wenigstens zwei selektiven Reagenzien entweder zur selektiven Abtrennung der anti-TPO-Autoantikörper und der anti-Tg-Autoantikörper vom Rest der flüssigen Probe oder zu deren selektiver Markierung vor oder nach ihrer Abtrennung von der flüssigen Phase eingesetzt werden.

3. Verfahren nach Anspruch 2, **dadurch gekennzeichnet, daß** das immundiagnostische Bestimmungsverfahren die Schritte umfaßt:
- Binden der in der flüssigen Probe vorhandenen Antikörper, einschließlich der zu bestimmenden Autoantikörper, mittels eines festphasengebundenen unspezifischen Binders an eine feste Phase,
- gemeinsame oder sequenzielle Zugabe von zwei verschiedenen Tracer-Reagenzien, die den gleichen Markierungsbestandteil enthalten, wobei eines der Tracer-Reagenzien spezifisch. alle gebundenen anti-TPO-Autoantikörper markiert und das andere der Tracer-Reagenzien spezifisch alle gebundenen anti-Tg-Autoantikörper markiert,
- Abtrennen der festen Phase von der flüssigen Reaktionsmischung und
- Messung der an die feste Phase gebundenen Markierung und Gewinnung eines Meßsignals, das die Summe der Mengen der anti-TPO-Autoantikörper und anti-Tg-Autoantikörper in der Probe repräsentiert.

4. Verfahren nach Anspruch 3, daß man als Tracer-Reagens zur Markierung von anti-TPO-Autoantikörpern einen cruden Extrakt aus humanen Schilddrüsen verwendet, der humane TPO in nativer, undenaturierter Form enthält, und in dem die humane TPO mittels markierter Antikörperfragmente selektiv markiert ist oder markiert werden kann, ohne daß ihre Bindungsfähigkeit gegenüber anti-TPO-Autoantikörpern beeinträchtigt ist.

5. Verfahren nach Anspruch 2, **dadurch gekennzeichnet, daß** das immundiagnostische Bestimmungsverfahren ein immunometrisches Sandwichverfahren, ein kompetitives Bestimmungsverfahren oder ein Verfahren ist, bei dem zur Trennung und/oder Markierung eine Doppelantikörpertechnik angewandt wird.

6. Verfahren nach einem der Ansprüche 1 bis 5, **dadurch gekennzeichnet, daß** man anhand eines durch Auswertung des Meßsignals erhaltenen positiven Meßergebnisses in Kombination mit der Krankheitsgeschichte und/oder den klinischen Symptomen des jeweiligen Patienten auf das Vorliegen einer Autoimmunthyreoiditis vom Typ Hashimoto, einer atrophischen Autoimmunthyreoiditis, eines primären Myxödems, einer asymptomatischen Thyreoiditis, einer postpartalen Thyreoiditis bzw. einer neonatalen Hypothyreose schließt.

## Claims

1. Method for the determination of thyroid autoantibodies in a biological sample obtained from a patient for the differential diagnosis of diseases which are associated with changes of the thyroid and/or disturbances of the normal thyroid function, which determination of the presence and/or amount of the thyroid autoantibodies in the sample is effected with the use of an immunodiagnostic assay method, **characterized in that** an immunodiagnostic assay method is used in which one test signal is obtained which represents the presence and overall amount of at least two antibodies, including anti-TPO autoantibodies and anti-Tg autoantibodies, in the sample.

2. Method according to Claim 1, **characterized in that** the immunodiagnostic assay method comprises the step of a reacting an optionally dilute liquid sample with at least two selective reagents, one of which selectively binds to anti-TPO autoantibodies and the other selectively binds to anti-Tg autoantibodies, the at least two selective reagents being used either for the selective separation of the anti-TPO autoantibodies and of the anti-Tg autoantibodies from the remainder of the liquid sample or for their selective labelling before or after their separation from the liquid phase.

3. Method according to Claim 2, **characterized in that** the immunodiagnostic assay method comprises the steps:
- binding of the antibodies present in the liquid sample, including the autoantibodies to be determined, to a solid phase by means of an nonspecific binder bound to a solid phase,
- combined or sequential addition of two different tracer reagents which contain the same labelling component, one of the tracer reagents specifically labelling all bound anti-TPO autoantibodies and the other tracer reagent specifically labelling all bound anti-Tg autoantibodies,
- separation of the solid phase from the liquid reaction mixture and
- measurement of the tracer bound to the solid phase and recovery of one test signal which represents the sum of the amounts of the anti-TPO autoantibodies and anti-Tg autoantibodies in the sample.

4. Method according to Claim 3, **characterized in that** the tracer reagent used for labelling anti-TPO autoantibodies is a crude extract of human thyroids which contains human TPO in native, undenatured form and in which the human TPO has been or can be selectively labelled by means of labelled antibody fragments without its binding capacity with respect to anti-TPO autoantibodies being impaired.

5. Method according to Claim 2, **characterized in that** the immunodiagnostic assay method is an immunometric sandwich method, a competitive assay method or a method in which a double antibody technique is used for the separation and/or labelling.

6. Method according to any of Claims 1 to 5, **characterized in that** the presence of an autoimmune thyroiditis of the Hashimoto type, of an atrophic autoimmune thyroiditis, of a primary myxoedema, of an asymptomatic thyroiditis, of a postpartal thyroiditis or of a neonatal hypothyroidism is concluded on the basis of a positive measured result obtained by evaluation of the test signal, in combination with the case history and/or the clinical symptoms of the respective patient.

## Revendications

1. Procédé pour la détermination d'auto-anticorps de la glande thyroïde dans un échantillon biologique prélevé chez un patient dans le cadre du diagnostic différentiel de maladies qui sont associées à des altérations de la glande thyroïde et/ou à des perturbations du fonctionnement normal de la glande thyroïde, la détermination de la présence et/ou de la quantité des auto-anticorps de la glande thyroïde dans l'échantillon étant réalisée en recourant à un procédé de détermination par immunodiagnostic,
**caractérisé en ce que** l'on applique un procédé de détermination par immunodiagnostic dans lequel on détecte un signal de mesure qui représente la somme de la présence et de la quantité d'au moins deux anticorps auxquels appartiennent les auto-anticorps anti-TPO et les auto-anticorps anti-Tg.

2. Procédé selon la revendication 1, **caractérisé en ce que** le procédé de détermination par immunodiagnostic comprend l'étape qui consiste à faire réagir un échantillon liquide, éventuellement dilué avec au moins deux réactifs sélectifs, dont l'un entre en liaison sélective avec les auto-anticorps anti-TPO et l'autre en liaison sélective avec les auto-anticorps anti-Tg, les réactifs sélectifs, au moins deux, étant utilisés soit pour la séparation sélective des auto-anticorps anti-TPO et des auto-anticorps anti-Tg du reste de l'échantillon liquide, soit pour leur marquage sélectif, avant ou après leur séparation de la phase liquide.

3. Procédé selon la revendication 2, **caractérisé en ce que** le procédé de détermination par immunodiagnostic comprend les étapes qui consistent à:
- lier les anticorps présents dans l'échantillon liquide, y compris les auto-anticorps à déterminer, à une phase solide au moyen d'un ligand non spécifique lié à une phase solide,
- ajouter en même temps ou successivement deux réactifs de marquage différents qui contiennent le même composant de marquage, l'un des réactifs de marquage marquant spécifiquement tous les auto-anticorps anti-TPO liés et l'autre des réactifs de marquage marquant spécifiquement tous les auto-anticorps anti-Tg liés,
- séparer la phase solide du mélange de réaction liquide et
- mesurer le marquage lié à la phase solide et détecter un signal de mesure qui représente la somme des quantités des auto-anticorps anti-TPO et des auto-anticorps anti-Tg dans l'échantillon.

4. Procédé selon la revendication 3, **caractérisé en ce que** pour le marquage des auto-anticorps anti-TPO, on utilise comme réactif de marquage un extrait brut de glande thyroïde humaine qui contient de la TPO humaine sous forme native et non dénaturée, et dans lequel la TPO humaine est marquée ou peut être marquée sélectivement au moyen de fragments d'anticorps marqués sans que sa capacité à se lier à des auto-anticorps anti-TPO soit influencée.

5. Procédé selon la revendication 2, **caractérisé en ce que** le procédé de détermination par immunodiagnostic est un procédé immunométrique sandwich, un procédé de détermination par compétition ou un procédé dans lequel on applique une technique à double anticorps pour la séparation et/ou le marquage.

6. Procédé selon l'une des revendications 1 à 5, **caractérisé en ce qu'**à l'aide d'un résultat de mesure positif obtenu par évaluation du signal de mesure, en combinaison avec l'historique de la maladie et/ou les symptômes cliniques du patient concerné, on tire des conclusions sur la présence d'une auto-immunothyroïdite de type Hashimoto, d'une auto-immunothyroïdite atrophique, d'un myxoedème primaire, d'une thyroïdite asymptomatique, d'une thyroïdite postpartale ou d'une hypothyréose néonatale.
